# EUROPEAN PATENT APPLICATION

(11) **EP 1 227 146 A1**
(43) Date of publication of application: **31.07.2002**
(21) Application number: 02001395.9
(22) Date of filing: 19.01.2002
(51) Int. Cl.: C12N 5/00, G01N 33/50, C07K 14/52

(54) **A method for creating endothelial cell dysfunction in cell culture**

(30) Priority: 29.01.2001 US 264780
(71) Applicant: WARNER-LAMBERT COMPANY, Morris Plains New Jersey 07950 (US)
(72) Inventor: Karathanasis, Sotirios Konstantinou, Saline, Michigan 48176 (US); Lin, Zhiwu, Ann Arbor, Michigan 48103 (US); Panek, Robert Lee, Ann Arbor, Michigan 48108 (US)
(74) Representative: Mansmann, Ivo

(57) **Abstract**

The proposed invention relates to a method for creating cell culture conditions which causes human endothelial cells in culture when exposed to a cytokine, preferably, TNFα for periods of longer than 5 days (chronic exposure) to become unresponsive to growth factors and other substances in serum. The cell culture assay system allows for screening for novel molecules that may correct endothelial dysfunction and will help identify genes that could be used to prevent or correct endothelial dysfunction with gene therapy.

## Description

### FIELD OF THE INVENTION

The present invention relates to methods for simulating endothelial cell dysfunction comprising culturing endothelial cells with a cytokine and a physiological protein matrix. In addition, the invention provides a method for testing substances for their ability to modulate angiogenesis and a method of determining whether an angiogenic entity such as endothelial cells will be responsive to anti-angiogenic or angiogenic therapy.

### BACKGROUND OF THE INVENTION

The vascular endothelium plays a central role in the preservation of normal vessel wall structure and function. In particular, endothelial cells control vascular permeability, vessel tone, coagulation, fibrinolysin, and inflammatory responses (Ross R. Atherosclerosis: An inflammatory disease. *New England*: *J. Med.* 1999;340:1 15-126). These functions are accomplished by production of a variety of biologically active substances. However, endothelial cell-mediated reactions may also lead to the development of pathological states within the vessel wall. Injury to endothelial cells (sometimes referred herein as EC) or endothelial cell dysfunction is considered one of the critical events in the development of atherosclerosis. Endothelial cell dysfunction is characterized by an impairment in capacity of the blood vessel to dilate and promote an increase in blood flow. This impairment reflects the enhanced metabolism of nitric oxide (NO) caused by the generation of reactive oxygen species (i.e., oxidative stress) such as superoxide anion (Toborek M., Kaiser S. Endothelial cell functions: Relationship to atherogenesis. *Basic Res. Cardiol.* 1999;94:295-314). In addition to its role as an endothelium derived vasodilator, NO appears to be an endogenous inhibitor of vascular smooth muscle cell growth and migration, NF-kB activity, and expression of proinflammatory molecules such as vascular cell adhesion molecule (VCAM-1) (Cines D.B., Pollak E.S., Buck C.A., Loscalzo J., Zimmerman G.A., McEver R.P., et al. Endothelial cells in physiology and in the pathophysiology of vascular disorders. *Blood* 1998;91:3527). Hence, the two characteristic features of endothelial dysfunction appear to result, in part from a decrease in NO bioactivity. This relative deficiency in NO activity predisposes vascular tissue to atherosclerotic lesion formation and ischemic injury. Furthermore, activation of endothelial cells plays a definitive role in many other processes, such as angiogenesis and allergic inflammation (Isner J.M. and Asahara T., Angiogenesis and vasculogenesis as therapeutic strategies for postnatal neovascularization. *J. Clin. Invest.* 1999;103:1231-1236; Weinberger M.S., Davidson T.M., Broide D.H., Differential expression of vascular cell adhesion molecule mRNA and protein in nasal mucosa in response to IL-1 or tumor necrosis factor. *J*. *Allergy Clin. Immunol.* 1996;97: 662). The altered properties of endothelial cells result from altered patterns of gene expression, for example, leukocyte invasion of the vessel wall depends on VCAM-1 and E-selectin expression by the endothelium (Ross R., Atherosclerosis―An inflammatory disease. *New Eng. J. Med.* 1999;340:115-126; Cines D.B., Supra., 1998). Impaired neovascularization during ischemic injury had been proposed to be due to reduced expression of certain growth factors and their receptors (i.e., VEGF and VEGF receptors) responsible for inducing endothelial proliferation, migration and capillary formation (Isner J.M. and Asahara T., Angiogenesis and vasculogenesis as therapeutic strategies for postnatal neovascularization. *J. Clin. Invest.* 1999;103:1231-1236).

Because chronic inflammatory cytokine-mediated endothelial activation has been proposed to participate in endothelial dysfunction, these effects were examined in vitro, using capillary tube formation as a measure of endothelial responsiveness. Furthermore, it was determined that the effects of short term and chronic TNFα treatment of endothelial cells on gene expression using gene chip profiling, to identify cytokine responsive genes which may be differentially upregulated or suppressed.

Endothelial cells form a single cell layer that lines all blood vessels and regulates exchanges between the blood stream and surrounding tissues. New blood vessels develop from the walls of existing small vessels by outgrowth of these endothelial cells, which have the capacity to form hollow capillary tubes even when isolated in culture. The process where new vessels originate as capillaries which sprout from existing small vessels is called angiogenesis. It can therefore be seen that angiogenesis plays a major role in normal tissue development and repair and in the progression of some pathological conditions (Isner J.M., Supra., 1999).

Once the vascular system is developed, endothelial cells of blood vessels normally remain quiescent and are not involved in vessel formation. If disease or injury occurs, the formation of new blood vessels can proceed, as in natural wound healing or be insufficient, as in chronic dermal ulcers, or non-operable peripheral vascular disease. Moreover, other factors contributing to impaired angiogenesis in animal models and patients alike, include aging, diabetes, and hypercholesterolemia (Isner J.M., Supra., 1999).The common denominator appears to be impaired endothelial function, manifest as reduced vasodilation and decreased production of nitric oxide. Chronic inflammatory cytokine-mediated endothelial activation has been proposed to participate in endothelial dysfunction in vivo. To better understand the mechanism(s) involved in impaired endothelial function, a cell culture model has been developed which mimics endothelial dysfunction using microcapillary tube formation as an indicator of endothelial integrity and responsiveness.

Current assays for angiogenesis are cumbersome, time consuming, and usually based on in vivo systems. The three most frequently used models are the rabbit corneal pocket, the hamster cheek pouch, and the chicken chorioallantoic membrane (CAM) assays. In each system, an angiogenic substances must be implanted in the cornea, cheek pouch, or the CAM in order to induce angiogenesis. All three assays suffer from the technical complexities associated with setting up the assay, including using live animals, and measuring the outcome. The rabbit corneal assay has the additional disadvantage of being ethically unacceptable in many research institutions.

Because of these disadvantages there is a great need for physiologically relevant, in vitro assays for angiogenesis, particularly human angiogenesis.

### SUMMARY OF THE INVENTION

The present invention relates to a method for determining angiogenesis, a method of screening substances for angiogenesis modulation activity, and a method of determining the appropriate treatment regime in the case of endothelial cellular-related disease and the like. In particular, the proposed invention relates to a method for creating cell culture conditions which causes human endothelial cells in culture when exposed to a cytokine, preferably, TNFα for periods of longer than 5 days (chronic exposure) to become unresponsive to growth factors and other substances in serum. In contrast to naive, untreated endothelial cells, chronic treatment of endothelial cells with TNFα when plated on a basement membrane protein matrix, do not differentiate into tube-like structures, resembling in vivo angiogenesis. Thus, chronic exposure to proinflammatory cytokines may render endothelium refractory to angiogenic stimuli and may be responsible for the lack of efficacy of vascular endothelial growth factor (VEGF) in causing therapeutic angiogenesis in patients (Isner J.M., Supra., 1999).

The current invention proposes a cell culture assay system that mimics endothelial "dysfunction" impairing angiogenic responses to serum factors. This system will allow screening for novel molecules that may correct endothelial dysfunction and will help identify genes that could be used to prevent or correct endothelial dysfunction with gene therapy.

The proposed invention will be used for identifying novel genes associated with impaired endothelial function. Moreover, in the proposed invention, the cell culture assay system is also amenable to screening for novel molecules that may correct endothelial dysfunction.

Chronic exposure of the vascular endothelium to inflammatory cytokines in response to vessel wall injury results in endothelial cell dysfunction contributing to the progression of atherosclerosis and ischemic syndromes. The persistent activation of the endothelium may induce the expression of genes, creating a positive feedback inflammatory cycle, leading to severe tissue damage. The described method for creating endothelial dysfunction in cell culture will be used as a source of tissue for identifying specific genes associated with impaired endothelial function. Those genes implicated in endothelial dysfunction will be developed as novel targets to discover and develop molecules that may correct endothelial dysfunction for treating atherosclerosis, acute and chronic coronary artery disease, hypertension, heart failure and for therapeutic angiogenesis.

### BRIEF DESCRIPTION OF THE FIGURES

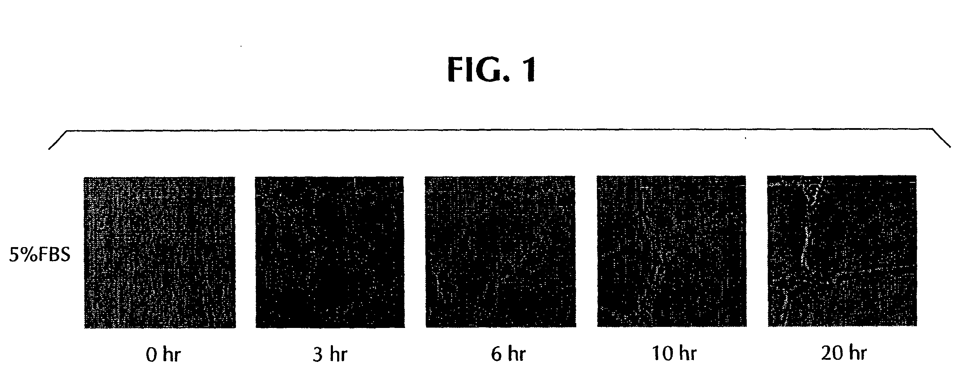

Figure 1 depicts a time course for development of tube-like structures following serum stimulation of Ea hy926 endothelial cells plated onto MATRIGEL®. At T=0 hour, cells were plated onto MATRIGEL® when they were nearly confluent. The cells assumed a cobblestone-like appearance. By 10 hours cells have begun to differentiate into distinct tube-like structures. Well defined tubes are evident at 20 hours post serum stimulation.

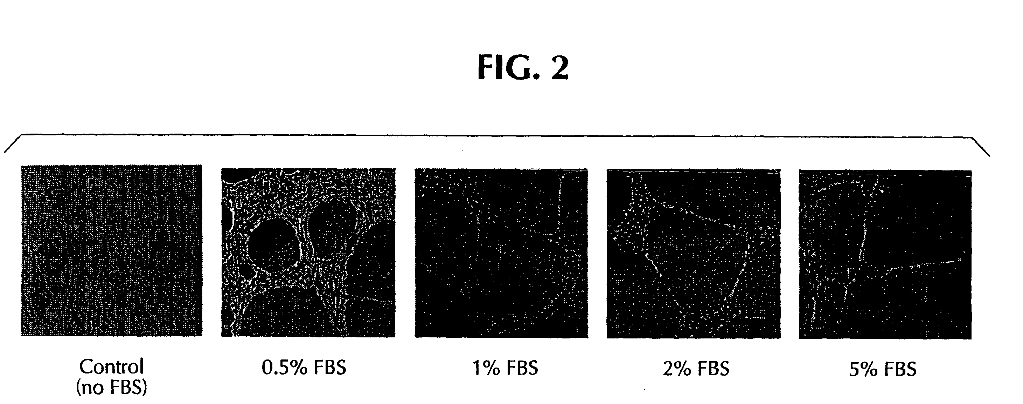

Figure 2 shows the effect of increasing serum concentration on development of endothelial tube-like structures. Ea hy926 endothelial cells plated onto MATRIGEL® form well defined tubules at serum concentrations of 2% and greater.

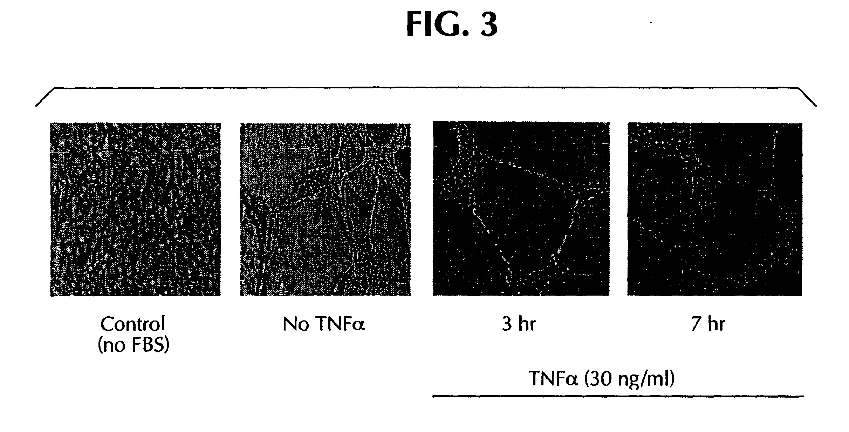

Figure 3 shows the effect of short term TNFα exposure on serum-stimulated tube formation. EA hy926 cells were first incubated on tissue culture plastic with TNFα (plus 5% FBS) for 3 or 7 hours, then plated on MATRIGEL® and incubated for 20 hours with 5% FBS to induce tube formation.

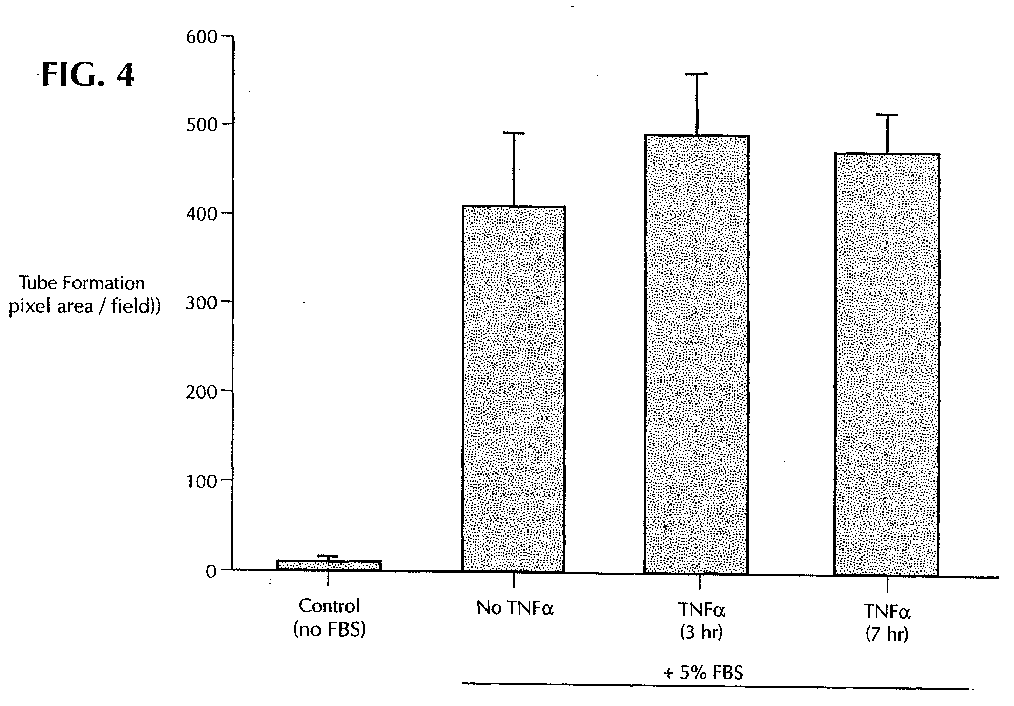

Figure 4 depicts quantitation of tube formation following short term TNFα exposure of EA hy926 cells. EA cells were first incubated on tissue culture plastic with TNFα (plus 5% FBS) or 3 or 7 hours, then plated on MATRIGEL® and incubated for 20 hours with 5% FBS to induce tube formation. Bars are the Mean ± SEM of 3 separate experiments.

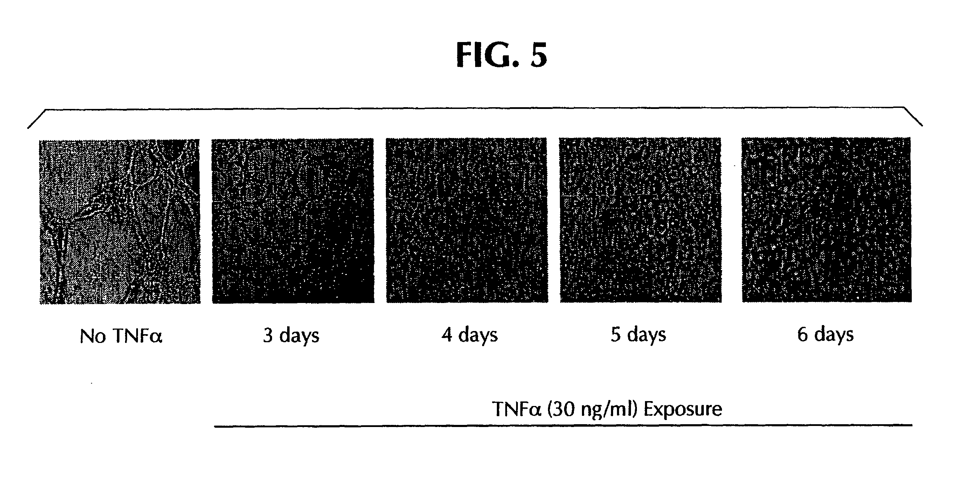

Figure 5 shows the effect of TNFα exposure on serum-stimulated tube formation. EA hy926 cells were first incubated on tissue culture plastic with TNFα (plus 5% FBS) for 3, 4, 5 or 6 days and then plated on MATRIGEL® in the presence of 5% FBS to induce tube formation. By Day 4 of TNFα treatment, cells no longer form tubes when stimulated with serum.

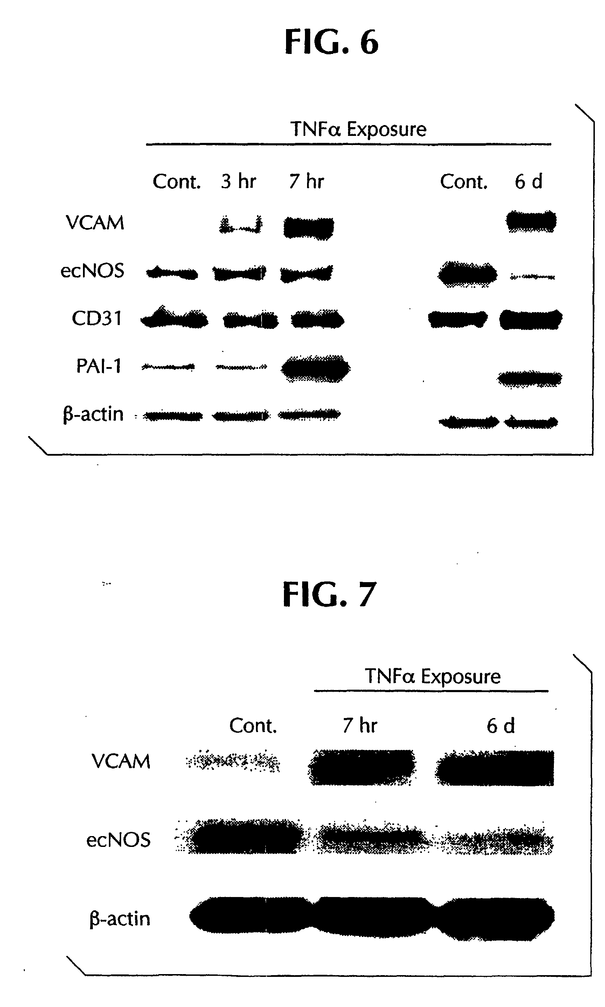

Figure 6 shows the effect of short and chronic TNFα exposure on the expression of VCAM, ecNOS, CD31, and PAI-1. EA hy926 cells were first incubated on tissue culture plastic with TNFα (plus 5% FBS) for 3 hours, 7 hours (short term), or 6 days (chronic). At the indicated times, cells were lysed and whole cell proteins subjected to gel electrophoresis and immunoblot analysis with appropriate antibodies as described in methods. β-actin was analyzed to verify equal protein loading.

Figure 7 shows the effect of short and chronic TNFα exposure on the mRNA expression of VCAM and ecNOS. EA hy926 cells were first incubated on tissue culture plastic with TNFα (plus 5% FBS) for 7 hours (short term) or 6 days (chronic). At the indicated times, cells were lysed and poly A RNA prepared as described in methods. β-actin was analyzed to verify equivalent RNA loading.

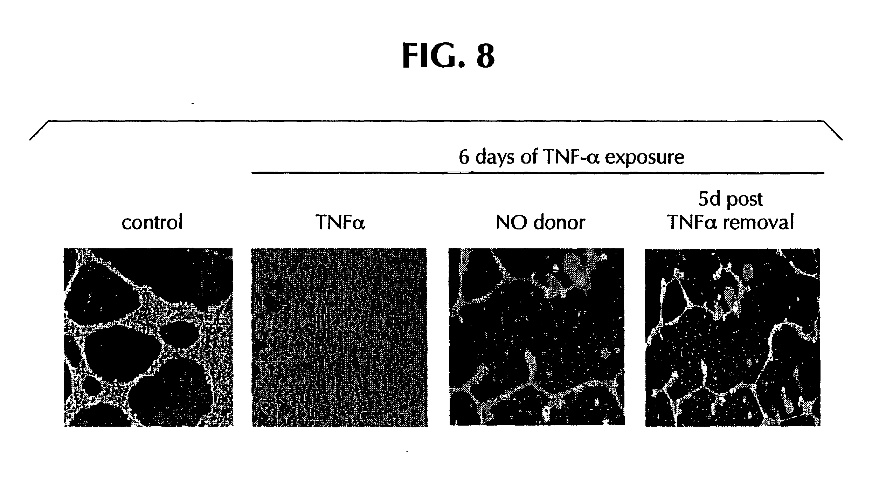

Figure 8 shows the effects of the NO donor SNAP or washing on chronic TNFα exposure to EA hy926 cells. Cells incubated on plastic with TNFα for 6 days fail to form tubes when plated on MATRIGEL® in the presence of 5% serum. Co-incubation of cells with TNFα and the NO donor (SNAP) for 6 days prior to plating on MATRIGEL® protects cells from TNFα induced dysfunction. Removal of TNFα by washing cells for 5 days also reverses TNFα-induced dysfunction.

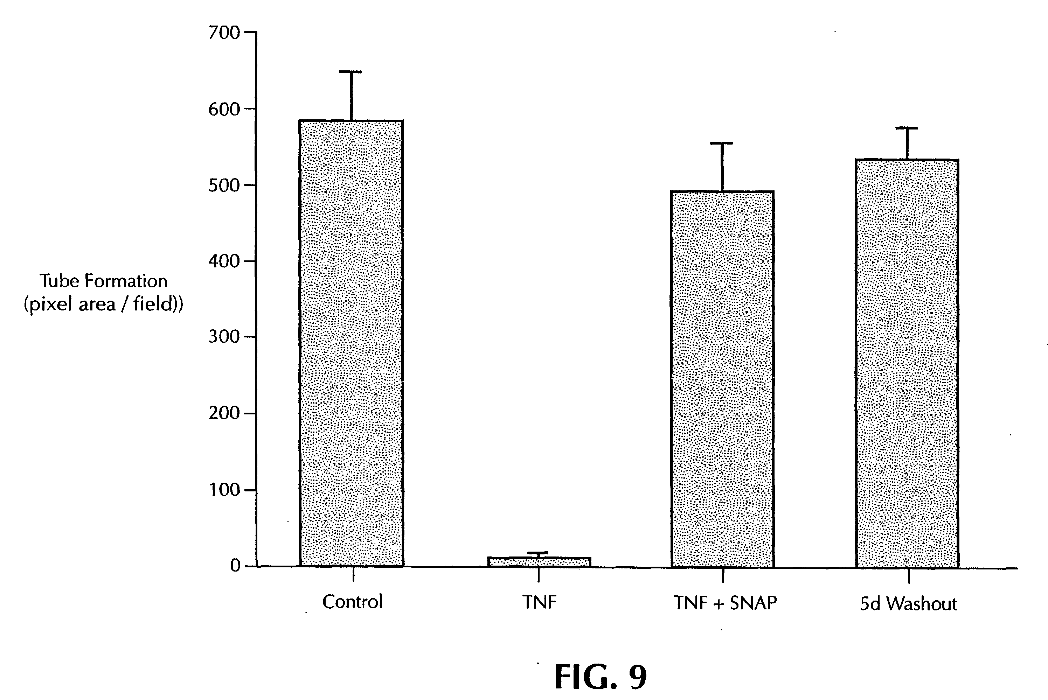

Figure 9 depicts the quantitation of tube formation following chronic TNFα exposure of EA hy926 cells in the presence of the NO donor SNAP or after a 5-day washout of cells that had been treated for 6 days with TNFα. Bars represent the mean ± SEM of three separate experiments.

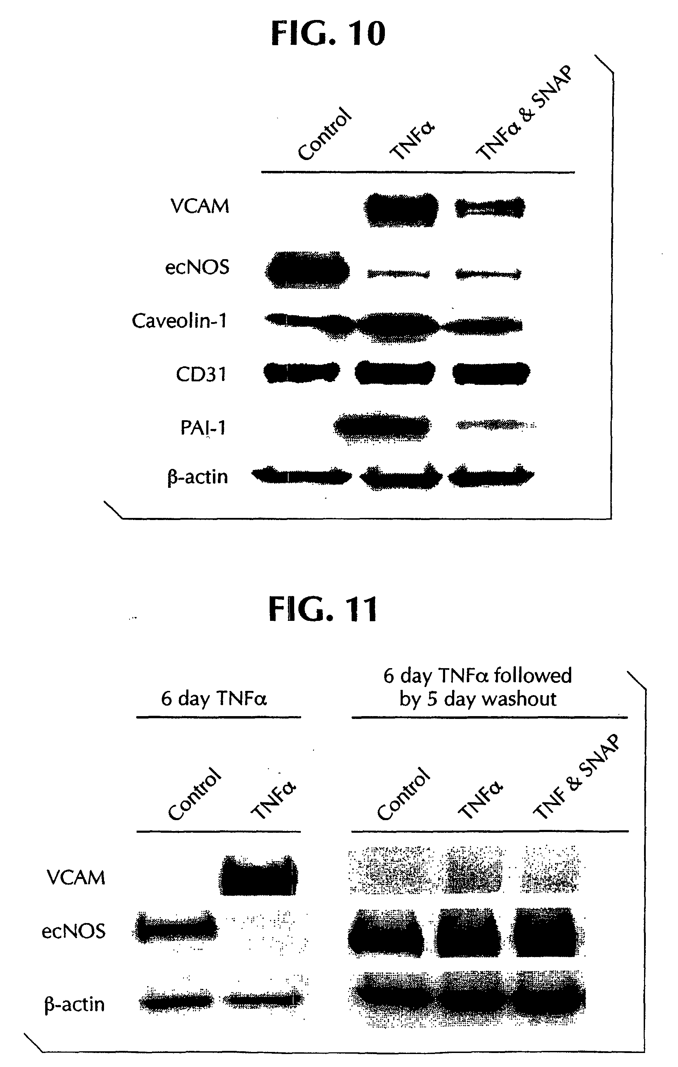

Figure 10 shows the effects of the NO donor SNAP on expression of the various endothelial marker proteins following chronic TNFα exposure to EA hy926 cells. Cells incubated on plastic with TNFα for 6 days in the presence of 5% serum increased VCAM, caveolin-1, CD31 and PAI-1 expression, but markedly decreased ecNOS. Co-incubation of cells with TNFα and the NO donor (SNAP) for 6 days reduces VCAM, caveolin- 1 and PAI-1 expression.

Figure 11 shows the effects of 5-day washout on the expression of VCAM and ecNOS following chronic TNFα exposure to EA hy926 cells. Cells incubated on plastic with TNFα for 6 days in the presence of 5% serum increased VCAM expression, but markedly decreased ecNOS. Five consecutive days of washing following TNFα treatment nullifies VCAM expression and restores ecNOS expression.

### DETAILED DESCRIPTION OF THE INVENTION

In a first aspect, the invention provides a method for simulating endothelial cell dysfunction comprising culturing endothelial cells together with a physiological protein matrix, serum, and a sufficient amount of cytokine to disrupt the normal growth response of the cells to the serum.

In a related aspect, the invention provides a method for determining angiogenesis comprising culturing endothelial cells together with a physiological protein matrix, serum, and a cytokine, and a suitable agent that allows growth of new vascular tissue, and examining the cells to determine whether new tissue has grown.

In another aspect, the invention provides a method for testing substances for angiogenesis modulation activity comprising culturing endothelial cells from a biological sample together with a physiological protein matrix, serum, cytokines, and at least one substance or compound to be analyzed for angiogenesis modulation activity for a time and under conditions sufficient to allow growth of new endothelial cells, examining the endothelial cells for new vascular tissue growth, and comparing the growth to that of a control.

In another aspect, the invention provides a kit comprising, in compartmentalized form, a first compartment or compartments adapted to receive a physiological matrix for culturing of endothelial cells, wherein the first compartment or compartments is optionally adapted to contain one or more physiological matrix precursors, a second compartment or compartments adapted to contain serum for addition to the physiological matrix to support growth of endothelial cells, a third compartment for cytokines to disrupt endothelial cell growth, and optionally frozen endothelial cells.

The term "physiological matrix" used herein means a material, support, or framework which simulates, mimics, or partially mimics a portion of the physiological environment within an organism which is necessary for angiogenesis to occur. Preferably the physiological matrix is fibrin, collagen, MATRIGEL®, (a basement membrane matrix composed of laminin, collagen IV, entactin, heparin sulfate protoglycan growth factors, matrix metalloproteinases and other components), or a similar material. Most preferably the matrix is fibrin.

The term "miniaturized scale" used herein means the use of small volumes of media. Suitable culture vessels for the miniaturized scale include microplates where liquid volumes of about 0.5 to 1 mL or less can be used as opposed to larger systems where typically tens of milliliters are used. Preferably, volumes of 0.5 to 1 mL or less are used in the cultures of the present invention. Preferably, exogenous factors which provide angiogenic stimuli are added to the medium.

The term "infrequently" used herein in relation to serum replacement refers to a nutrient replacement occurring less frequently than every second day. Preferably nutrient replacement is twice a week, more preferably once every 4 days, in the method of the present invention.

Endothelial cells used in the methods may be derived from various sources. Preferably, the endothelial cells are freshly isolated. Alternatively, frozen endothelial cells may be used, for example, in kits. Preferably the endothelial cells used in the method is derived from human tissue, for example, human tissue which is readily available such as placentas and solid tumors. It will, of course, be appreciated that the method of the invention could also be used to assay angiogenesis in non-human endothelial cells.

The nutrients are preferably supplied in a liquid medium such as Medium 199 optionally containing 20% fetal calf serum and may also contain, in addition, antibiotics to inhibit the growth of microorganisms. Alternatively the medium may be a substantially serum free medium. The term "substantially serum free" used herein means that whole serum is absent, and the medium has no serum constituents or a minimal number of constituents from serum or other sources which are necessary for angiogenesis. Those skilled in the art will be familiar with the appropriate media.

In the method, the endothelial cells are cultured under suitable conditions to allow the growth of new vascular tissue (angiogenesis), but for the presence of cytokines. Those skilled in the art will be familiar with the various suitable conditions which may be used to culture endothelial cells. Preferably, the endothelial cells are grown at about 37°C.

Examination of the endothelial cells may be carried out by any convenient means known to those skilled in the art. Preferably examination of the endothelial cells is carried out by bright field or phase contrast light microscopy. This may be done using an inverted phase contrast microscope. The responses of the cells (angiogenic or otherwise) can be quantified manually or by computer-based image analysis of photographs, video images, or digital images of the cultures. Preferably the responses are quantified by automated means such as by the NIH IMAGE program. Such quantification provides rapid and accurate assessment of the responses. In addition, such an assay system is particularly well-suited to screening inhibitors or enhancers of human angiogenesis. Those skilled in the art will be familiar with the various ways of quantifying the responses of the cells.

The term "screening" refers to testing or assaying the substance(s) or compound(s) for its ability to modulate endothelial cell function, including angiogenesis.

The term "angiogenesis modulation" refers to the ability of a substance or compound to modulate or change normal angiogenic activity of the endothelial cells and includes inhibition and enhancement of angiogenic activity. The method may be used to test the compounds or substances, which are possible angiogenesis inhibitors or possible angiogenesis promoters.

The term "biological sample" refers to any sample which is ultimately derived from an animal tissue, where it is desirable to test whether a substance or compound has angiogenesis modulation activity for that particular tissue and/or animal species. Preferably, the biological sample is derived from human tissue.

Any substance, compound, or combination of substances or compounds which are suspected of angiogenesis modulation activity may be screened by the method. This includes purified preparations of compounds and various extracts such as plant or animal tissue extracts or may be from a microorganism. Accordingly, such substances may have to be brought into a suitable form for administration to the endothelial cells. Those skilled in the art will be familiar with various methods for bringing such substances into suitable form for administration. As mentioned above, endothelial cells may be derived from venular or arterial origin. Preferably, the cells are used both for the control and cultures being screened with potential angiogenesis modulation activity.

Preferably, when the method is used to test compounds for angiogenesis correction or enhancement, the medium is substantially serum free (as previously defined).

The proposed invention employs a human endothelial cell line, referred to as EA hy926 used as a model cell line. EA hy926 was originally generated by C-J.S. Edgell, University of North Carolina, NC (Edgell C.J.S., McDonald C.C., Graham J.B. Permanent cell line expressing factor VIII related antigen established by hybridization. *Proc. Natl. Acad. Sci. USA* 1993;80:3734-3737). EA hy926 is a continuous, clonable, human cell line that displays features characteristic of vascular endothelial cells. An in vitro assay system which models angiogenesis in vivo was used to measure endothelial cell responses (Bauer J., Margolis M., Schreiner C., Edgell C.J., Azizkhan J., Lazarowski E., Juliano R.L. In vitro model of angiogenesis using a human endothelium-derived permanent cell line. *J. Cell. Physiol.* 1992;153:437-449). Angiogenesis can be divided into three phases: proliferation, migration, and endothelial cell differentiation. Current in vitro assay systems which depend on provision of a protein matrix effectively measure the ability of endothelial cells to differentiate. Assay systems measuring differentiation involve the formation of cord-like or tube-like structures by endothelial cells. All such systems depend on supplying the cells with exogenous basement membrane proteins on which the cells migrate to form tubules. Cell differentiation and tube formation occurs over a short time period, usually 16 to 24 hours to give rise to three dimensional tube-like structures. In addition to the matrix proteins, this system requires the provision of serum-derived growth factors to produce acceptable tube formation. The time scale over which tubes are formed provides an excellent test for inhibition of endothelial differentiation.

According to an aspect of the invention, EA hy926 (EA) cells are initially grown in standard endothelial cell culture medium consisting of MCDB 131 (Clonetics Corp.) supplemented with 10 fetal bovine serum (FBS) (Hyclone laboratories), 100 U/mL penicillin/streptomycin, 50 µg/mL gentamicin, and other endothelial growth supplements (EGM bullet kit, Clonetics Corp.). Cells used for experiments were seeded into 75 cm² culture flasks with or without human TNFα (30 ng/mL) containing the above growth supplements plus 5% FBS. Cells were grown in these conditions for 6 to 7 days. EA cells are then dispersed by trypsinization, counted, suspended in EGM media containing 5% FBS, and plated onto the surface of a MATRIGEL® basement membrane protein matrix (0.5 mL/well, dispensed into 24-well tissue culture plates according to the manufacturer's instructions) at a density of 5 × 10⁵ cells per well (24 well plate). The cells were then placed in a CO₂ incubator at 37°C and observed over a 16- to 24-hour period for cell differentiation into tube-like structures resembling capillaries. The wells are then imaged with a Nikon inverted phase contrast microscope (40X objective) using a Sony CCD3 video camera.

Preferably, the assay method is performed on a miniaturized scale and comprises steps of:
(a) Setting up endothelial cell cultures containing a suitable culture medium for sustaining growth of cells therein.
(b) Setting up an identical set of endothelial cell cultures containing a suitable culture medium plus a set concentration of TNFα (usually 10 ng-30 ng/mL).
(c) Incubating cell cultures over a period of 6 to 7 days with or without TNFα, and refreshing the culture medium with TNFα every second day.
(d) Removing all the growth medium after Days 6 to 7, washing cells in PBS, then dispersing cells by trypsinization and plating cells onto the surface of a basement membrane protein matrix, preferably, MATRIGEL® basement membrane matrix.
(e) Adding fresh growth medium to the cells on the protein matrix, and monitoring the progress of the cells for the appearance of tube-like structures is over a period of 16 to 24 hours.
(f) Cell cultures, having been treated over a period of 6 to 7 days with TNFα, will not form tube like structures when plated onto MATRIGEL® basement membrane matrix. This lack of endothelial cell differentiation response is defined as endothelial dysfunction.

A preferred embodiment of the invention is illustrated in Figure 2 where normal EA hy926 cells plated onto MATRIGEL® membrane protein matrix will differentiate into tube-like structures in the presence of serum, a process which is both time- and serum-concentration dependent. In contrast, Figures 3 through 5 show when cells are exposed to TNFα for greater than 3 days in the culture flask containing growth medium (serum) and then plated onto MATRIGEL®; the cells no longer form tubes when stimulated with serum. However, the same chronic exposure of endothelial cells with TNFα together with an agent referred to as a nitric oxide donor (NO donor) can reverse or correct the endothelial dysfunction, whereby the cells are capable of differentiating into tube-like structures when plated on a basement membrane protein matrix (Figure 8). This chronic treatment with TNFα does not result in cell death, because removal of TNF followed by replacement of fresh serum containing medium for 5 days results in normal endothelial responsiveness, associated with formation of tube-like structures when plated on a basement membrane protein matrix (Figures 8 and 9).

The present invention is useful to identify specific genes associated with impaired endothelial function and develop targets that will correct endothelial dysfunction and the disease states associated therewith (see Table 1). Angiogenic disease states associated with endothelial cells include tissue development and wound healing. Other angiogenesis-dependent diseases include, but are not limited to, the development of solid tumors, proliferative retinopathies, and rheumatoid arthritis.

The in vitro assay of the present invention provides an angiogenic response which simulates a normal physiological response. The overall cost of the assay (i.e., labor costs, media, and tissue flask expenses) is dramatically reduced, and the assay allows rapid examination and quantification. In addition, the assay in all of its embodiments is ethically acceptable because it avoids the use of live animals. Furthermore, it provides direct information about the effects of particular angiogenic modulating substances on a particular species because vascular tissue from that species can be used in the assay. For example, the assay can directly determine whether a particular substance has angiogenic modulating ability in humans since human tissue may be used in the assay.

### EXAMPLE 1

### Materials and Methods

EBM medium and its supplement were purchased from Clonetics. MATRIGEL® was purchased from Collaborative Biomedical Products (40234C). Anti-human VCAM-1 antibody was purchased from R&D System (Cat. BBA19). Anti-Human Caveolin antibody was purchased from Upstate Biotechnology (Cat. 06-591). Anti-ECNOS mAb was purchased from Transduction Laboratories (Cat. N30020). CD31 antibody was purchased from ENDOGEN (Code MA-3107). Actin antibody was purchased from Santa Cruz. Biotechnology (Cat. SC-1616). Recombined human TNFα was purchased from R&D System (Cat. 210-TA). Nitric oxide donor/S-Nitroso-N-acetyl-D, L-penicillamine (SNAP), was purchased from Toronto Research Chemicals Inc. (Cat. M56500). Phosphate-buffered saline for Western blotting was ordered from Life Technologies (Cat. 21300-058). ECL kit with secondary antibodies was purchased from Amersham Pharmacia Biotech (Cat. RPN 2108). One-Step™ RT-PCR System was purchased from Life Technologies (Cat. 10928-018). The primers for RT-PCR were either synthesized by Life Technologies or directly ordered from R&D System (BPR-157 for hV-CAM and BPR-188 for h β-Actin). The synthesized primers by Life Technologies were human e NOS and human Caveolin (Sense strand primer for e NOS: CTA AGC AGG CCT GGC GCA AC, anti-sense fore NOS: GCA GCA GCA GGG GCA GCA CG. Sense strand primer for Caveolin: GAT CTC AGG CGT TCA GGC CC, anti-sense for Caveolin: ACC CAC TAT TCA GTA TCC AT). TA Cloning Kit was ordered from Invitrogen (Cat. K2050-01). The reagents for Northern analysis were ordered from Boehringer Mannheim (Cat. 1636090, 1093274, 1655884, 1603558, 1585762). Total RNA and mRNA isolation kits were purchased from Qiagen Inc. (Cat. 75124, 70042). The human endothelial cell line EA hy926 was used for these studies. EA hy926 was originally generated by C-J.S. Edgell, University of North Carolina, NC (Edgell et al., *PNAS*, 1983;80). EA hy926 is a continuous, clonable, human cell line that displays features characteristic of vascular endothelial cells. EA hy926 (EA) cells were initial grown in standard endothelial cell culture medium consisting of MCDB 131 (Clonetics Corp.), supplemented with 10 fetal bovine serum (FBS) (Hyclone Laboratories), 100 U/mL penicillin/streptomycin, 50 µg/mL gentamycin, and other endothelial growth supplements (EGM bullet kit, Clonetics, Corp.). Cells used for experiments were seeded into 75 cm² culture flasks with or without human TNFα (30 ng/mL) containing the above growth supplements plus 10 FBS. Cells were grown in these conditions for 5 to 6 days. EA cells were then dispersed by trypsinization, counted, suspended in EGM media containing 10% FBS, and plated onto the surface of a MATRIGEL® basement membrane matrix (0.5 mL/well, dispensed into 24-well tissue culture plates according to the manufacturer's instructions) at a density of 5 × 105 cells per well (24-well plate). The cells were then placed in a CO2 incubator at 37°C and observed over a 16- to 24-hour period for formation of tube-like structures. The wells were then imaged with a Nikon inverted phase contrast microscope (40X objective) using a Sony CCD3 video camera.

### Tube Formation Assay

MATRIGEL® (0.5 mL/mL) was dispensed into 24-well tissue culture plates according to the manufacturer's instructions. EA hy296 cells were trypsinized from flasks and counted with Coulter Z-2 cell counter. The counted cells were pelted down by centrifugation at RCF 220 for 10 minutes and suspended with serum-free EBM medium at the concentration of 2×105 cells/mL. One milliliter of the suspended cells was accurately aliquoted into each well of 24-well tissue culture plates. The cells were placed in a tissue culture incubator for 2 to 4 hours before replacing with desired medium. From this time point on, the cells were periodically observed and photographed using a Sony CCD camera connected to a Nikon Diaphot inverted microscope. The quantity of tubes formed were analyzed with NIHIMAGE. For TNF treatment, EA hy926 was incubated with 5% stripped fetal bovine serum and 30 ng/mL recombined human TNFα. When such TNFα treated cells were used for tube formation assay, 5×10⁴ cells were plated into each well.

### Total RNA and mRNA Isolation

QIAGEN manufactured RNA isolation and purification kits were used for RNA isolation. In summary, EA hy926, either treated with human TNF or non-treated, were trypsinized from flasks. 10⁷ cells were pooled together and centrifuged at RCF 220 for 10 minutes. The cell pellets were suspend ed in lysis buffer. The RNAs were purified using a silica-gel membrane made spin column according to the instructions provided with the kit. mRNA was made using Oligotex mRNA Midi Kit produced by Qiagen. In general, 500 µg total RNA for each sample was mixed with Oligotex containing a dT oligomer coupled to a solid-phase matrix. After several washes and spins, mRNA, which survives such washing by binding to Oligotex with dT:A hybridization, was eluted with low salt solution according to the instructions. RNA quantitation was measured with Beckman DU 640 Spectrophotometer.

### CDNA Cloning of ecNOS, VCAM, and β-actin

RT-PCR technique was employed to produce cDNA fragments with specific oligos designed within the coding sequences. After purification the cDNA fragments, which were about 400 bp in size, were cloned into pCR II vector provided with Invitrogen kit, followed by transformation, plasmid preparation, restriction enzymes analysis, and sequencing. The sequencing proved clones were amplified and used for Northern analysis probes.

### Northern Analysis

2 µg mRNAs were electrophoretically separated in formaldehyde agarose gel and transferred to nylon membrane (Nytran Plus) purchased from Schleicher & Schuell. The RNA was pre-hybridized with DIG EASY HYB solution from BMB at 50°C for 2 hours. The probes for eNOS, V-CAM, and β-actin made by PCR with PCR DIG Probe Synthesis Kit purchased from Boehringer Mannheim were purified by electrophoresis in agarose gel and 25 ng of each probe was used in 15 mL of hybridization buffer. The incubation was carried out overnight at 50°C for hybridization. After hybridization and stringency washes, the membranes were pre-incubated for 30 minutes with the blocking buffer from BMB kit at room temperature. The incubation of the membranes with anti-DIG-AP conjugate at 1:10,000 dilution in blocking buffer was carried out for another 30 minutes, then followed by washing, incubation with CSPO from BMB, and developing the signals.

### Western Blotting Analysis

The whole cell lysates were generated by suspending cell pellets with protein lysis buffer containing 10 mM Tris.CI (pH 7.6), 1 mM EDTA (pH 8.0), 100 mM NaCI, 1 µg/mL aprotinin, and 100 µg/mL phenylmethylsulfonyl fluoride. The lysates were sonicated for 15 seconds at output control level 4 with Sonifier 450 model manufactured by VWR Scientific products. Fifty microgram lysates protein for each sample was electrophoretically separated in 8% to 16% Tris-Glycine Gel and transferred to a nitrocellulose membrane. The proteins on the membrane were stained with PONCEAS solution to check if the proteins loading and transfer were even. The membranes were separately incubated with 5% dry milk Phosphate-buffered saline (PBS) at room temperature for 2 hours and followed by overnight incubation at 4°C with fresh 5% dry milk PBS containing primary antibodies at 1:2000 dilution. The peroxidase conjugated secondary antibodies was diluted at 1:4000 dilution with 5% PBS buffer. The incubation with secondary antibody was carried out at room temperature for 1 hour. The membranes were then subject to staining with ECL solution and exposure to films.

### Sample Preparation and Hybridization on Affymetrix GeneChips

Total RNA was isolated with Trizol from EA cells that had been stimulated for either 6 hours or 6 days with 30 ng/mL of TNFα. Control cells were treated with vehicle for the same times and RNA isolated as above. Transcript integrity was monitored using denaturing agarose gel electrophoresis in 1X MOPS. Biotinylated target RNA was prepared from 15 µg of total RNA using the Affymetrix protocol. Briefly, double-stranded cDNA was prepared from the RNA template using a modified oligo-dT primer containing a 5' T7 RNA polymerase promoter sequence and the Superscript Choice System for cDNA Synthesis (Life Technologies Inc., Gaithersburg, MD). Following phenolchloroform extraction and ethanol precipitation, one-half of the cDNA reaction (0.5-1.0 µg) was used as template in an in vitro transcription reaction containing T7 RNA polymerase, a mixture of unlabeled ATP, CTP, GTP, and UTP, and biotin-11-CTP and biotin-16-UTP (BioArray High Yield Kit, ENZO, Inc.). The resulting biotinylated-cRNA "target" was purified on an affinity resin (RNeasy, Qiagen) and quantified using the convention that 1 O.D. Two-hundred sixty corresponds to 40 µg/mL of RNA. Typical yields ranged from 50 to 100 µg with transcript sizes between 3.0 to 0.25 nucleotides as determined by gel electrophoresis. Fifteen micrograms of biotinylated cRNA was randomly fragmented to an average size of 50 nucleotides by incubating at 94°C for 35 minutes in 40 mM TRIS-acetate, pH 8.1, 100 mM potassium acetate, and 30 mM magnesium acetate. The fragmented cRNA was hybridized in a solution containing 100 mM MES, 1 M [Na⁺], 20 mM EDTA, 0.01% TWEEN 20, 50 pM of Control Oligonucleotide B2 (Affymetrix, Inc.), 0.1 mg/mL of sonicated herring sperm DNA, and 0.5 mg/mL BSA for 16 hours at 45°C on a Human 6800 Affymetrix GeneChip. Each hybridization included a mixture of four bacterial biotinylated-RNA transcripts (BioB, BioC, BioD, and cre) spiked at 1.5, 5, 25, and 100 pM, respectively. The hybridization reactions were processed and scanned according to the standard Affymetrix protocols.

### Results

### Characterization of in vitro angiogenesis; Effects of TNFα on Tubular morphogenesis of endothelial cells

TNFα has multiple functions in a variety of cell types and plays an important role in inflammatory processes in the vessel wall including effects on endothelial function. To investigate the short-term versus chronic effects of cytokines on endothelial cell activation, a 3-dimensional cell culture system was utilized that resembles angiogenesis. Whereas it is likely that this in vitro process of MATRIGEL®-induced morphological re-organization is not precisely the same as the generation of new capillaries seen during in vivo angiogenesis, it offers a convenient model to study some of the biochemical and molecular events associated with angiogenesis.

Figure 1 shows EA hy926 (EA) cells plated onto a matrigel membrane protein matrix will differentiate into tube-like structures. Cells initially assumed a cobblestone-like appearance when applied to matrigel. However, by 20 hours after plating, EA cells had differentiated into distinct tube-like structures resembling in vivo angiogenesis, a process which was serum dependent. Figure 2 shows that in control cells without serum, tube formation could not be observed after 20 hours, however, EA cells formed well-defined tubes at serum concentrations of 2% or greater. Figure 3 shows EA cells when exposed to TNFα for a short time (i.e., 3-7 hours) in the culture flask containing growth medium (5% FBS) and then plated onto matrigel, form tubes when stimulated with serum. Short-term TNFα treatment did not alter the quantity or quality of tubular structures (Figures 3 and 4). In contrast, Figure 5 shows when EA cells were exposed to TNFα for greater than 3 days in the culture flask containing growth medium (5% FBS) and then equal numbers of cells plated onto matrigel, the cells no longer formed tubes. This effect was not associated with a loss of cell viability (determined by trypan blue staining, data not shown). Figure 6 shows western blot analysis of EA cells incubated with 10 ng/mL TNFα (in 5% FBS) for 3 hours, 7 hours, or 6 days (chronic exposure). Analysis of cell lysates showed that short exposure increased VCAM-1 and PAI-1 but not ecNOS or CD31. In contrast, 6-day TNFα treatment increased expression of VCAM-1 and PAI-1 and markedly decreased ecNOS, consistent with the inability of the EA cells to form tubes (Figure 5). Short and chronic TNFα exposure produced a similar increase in VCAM-1 mRNA expression, however, ecNOS levels were reduced following 7-hour treatment and completely inhibited following 6-day TNFα treatment (Figure 8). Nitric oxide (NO) has been reported to improve endothelial function. Therefore, EA cells were co-incubated with TNFα (10 ng/mL) and the NO donor, SNAP (200 µM) for 6 days prior to plating on MATRIGEL® to determine whether cells could be protected from TNFα-induced dysfunction. Figure 8 shows tube formation when SNAP is present during TNFα exposure to EA cells. In addition, removal of TNFα (after 6-day treatment) by rinsing cells for 5 days followed by plating on matrigel resulted in recovery of tube formation (Figure 8). Quantitation of tube structures showed similar increases in number of tubes for control compared to SNAP-treated or rinsed cultures compared to the chronic TNFα-treated cells (Figure 9). Figure 10 shows a biochemical analysis of the effects of the NO donor, SNAP on expression of endothelial marker proteins following 6-day TNFα exposure to EA cells. Chronic TNFα treatment upregulated expression of VCAM-1, PAI-1, caveolin-1, and CD31, but markedly decreased ecNOS. Co-incubation of EA cells with TNFα and SNAP (200 µM) for 6 days reduced VCAM-1, caveolin-1, and PAI-1 expression toward control levels. In addition, we assessed the effect of a 5-day washout of TNFα from EA cultures on the expression of VCAM-1 and ecNOS protein. Figure 11 shows that 5 consecutive days of rinsing EA cells following the 6-day TNFαtreatment restored ecNOS expression to control levels and reduced VCAM-1 to background. The presence of SNAP (200 µM), together with TNFα (6 day exposure), did not affect the recovery of ecNOS expression or the loss of VCAM-1 expression as a result of the washout.

### Gene Expression profiling studies

EA cells incubated with 10 ng/mL TNFα (with 5% FBS) for 6 hours (short-term) or 6 days (chronic exposure) were evaluated for identification of cytokine-responsive endothelial genes. Analysis of RNA samples via affymetrix gene chip (6800 genes) showed that short exposure with TNFα increased VCAM-1 (32-fold above control) but did not affect ecNOS expression (Table 1), consistent with protein and mRNA expression observed for these genes (Figures 6 and 7). However, 6 day TNFα treatment increased expression of VCAM-1 about 76-fold over its respective control, twice the increase compared to short-term TNFα treatment. In addition, ecNOS expression was reduced 4-fold from control, consistent with the western and northern blot data (Figures 6 and 7). Table 1 shows short-term TNFα exposure induced the expression of a number of proinflammatory (proatherogenic) genes from 2.7- to 4.7- fold including vitronectin, vitronectin receptor αV subunit, CD-44, MT-MMP, and type IV collagenase, whereas VCAM-1 expression was markedly upregulated (32-fold). Chronic TNFα exposure caused even greater increases in expression of these genes (6.8-75.8 fold). Similar results were observed for leukocyte trafficking genes (ie, MCP1, IL-8). TNFα exposure to EA cells also resulted in increased expression (4.3-17.7 fold) of a number of protective genes including, superoxide dismutase and anti-apoptotic genes including, A-20, hiap-1, TRAIL, and CD95.

The results from the present study demonstrate that persistent endothelial activation leading to dysfunction can be mimicked in vitro and corroborated with alterations in cytokine responsive genes. Although supplying nitric oxide proved to be one way to restore impaired endothelial function, at least in this model, gene profiling experiments revealed a number of additional candidate genes whose modulation could likely affect endothelial responsiveness. These findings combined with data from other differential screening procedures on endothelial cells in processes such as endothelial differentiation (Shima D.T., Saunders K.B., Gougos A., D'Amore P.A. Alterations in gene expression associated with changes in the state of endothelial differentiation. *Differentiation,* 1995;58:217-226), proliferation and migration (Topper J.N., Cai J., Falb D., Gimbrone M.A. Identification of vascular endothelial genes differentially responsive to fluid mechanical stimuli: COX-2, Mn-SOD ande-NOS are selectively up-regulated by steady laminar shear stress. *Proc. Natl. Acad. Sci.* USA. 1996;93:10417), and hemodynamic forces (Kozian D.H., Augustin H.G. Rapid identification of differentially expressed cell genes by RNA display. *Biochem. Biophys. Res.* Commun. 1995;209:1068), will yield detailed information on what distinguishes an activated or dysfunctional endothelial cell from a healthy, responsive cell. This information will be needed to better understand the pathophysiology of the atherosclerotic vessel wall as well as impaired angiogenesis or revascularization during ischemic injury.

**Table 1.**

| Effect of Short-Term (6 hr) Versus Chronic (6 Day) Cytokine Stimulation of EA.hyb926 Endothelial Cells on Gene Expression by Gene Chip Profiling | | | |
|---|---|---|---|
| Gene Product | Accession Number | Short- (6 hr Tx) Fold Increase | Chronic- (6 day Tx) Fold Increase |
| *Cell Proliferation*/*Migration* | | | |
| ecNOS (NOS 3) | M93718 | nc | -3.9 |
| VEGF C | U43142 | 7.8 | 27.1 |
| | | | |

| *Apoptosis* | | | |
|---|---|---|---|
| A20: TNFα-inducible transcription factor | M59465 | 6.5 | 6.6 |
| Hiapl | U45878 | 17.7 | 6.9 |
| TRAIL | U37518 | nc | 4.8 |
| CD95 (Fas) | X83492 | nc | 4.3 |
| | | | |

| *Signaling*/*Regulation of Transcription* | | | |
|---|---|---|---|
| NFkB1 (p105) | M58603 | 4.4 | 2.9 |
| HIF-1 α | U22431 | 4.6 | Nc |
| | | | |

| *Leukocyte Trafficking* | | | |
|---|---|---|---|
| MCP-1 | HG4069 | 4.5 | 8.7 |
| MCP-2 | X99886 | nc | 131.7 |
| MCP-3 | X72308 | nc | 19.9 |
| Interleukin-8 | M28130 | 4.6 | 32.6 |
| RANTES | M21121 | 39.5 | 62.8 |
| GM-CSF | M13207 | 23.0 | 37.1 |
| Prointerleukin-1β | X04500 | 42.2 | 213.1 |
| Macrophage inflammatory protein2-β | X53800 | 11.2 | 40.6 |
| | | | |

| *Oxidative Damage Protection* | | | |
|---|---|---|---|
| Manganese superoxide dismutase | X07834 | 13.1 | 63.4 |
| | | | |

| *Matrix and Adhesion molecules* | | | |
|---|---|---|---|
| Integrin, αV subunit (vitronectin receptor) | X68742 | 4.3 | 6.8 |
| Type IV collagenase | J05070 | 4.7 | 27.2 |
| Vitronectin | M14648 | 4.3 | 6.8 |
| VCAM-1 | M60335 | 32.3 | 75.8 |
| MT-MMP | X83535 | 4.3 | 26.1 |
| Von Willebrand factor | M10321 | nc | -8.3 |
| TNF-Inducible, CD44 adhesion receptor | M31165 | 2.7 | 37.1 |

| | | | |
|---|---|---|---|
| nc = no change from vehicle treated. | | | |

All publications mentioned in the specification are herein incorporated by reference to the same extent as if each independent publication was specifically and individually indicated to be incorporated by reference.

While the invention has been described in connection with specific embodiments thereof, it will be understood that it is capable of further modifications. The application is intended to cover any variations, uses, or adaptations following, in general, the principles of the invention and including such departures from the present disclosure within known or customary practice within the art to which the invention pertains and may be applied to the essential features herein before set forth.

## Claims

1. A method for simulating endothelial cell dysfunction, said method comprising culturing endothelial cells together with a physiological protein matrix, growth media, and an effective amount of at least one proinflammatory cytokine sufficient to disrupt normal response of the endothelial cells to the growth media.

2. A method according to Claim 1, wherein the cytokine is tumor necrosis factor α (TNFα).

3. A method according to Claim 1, wherein the physiological protein matrix is a basement membrane protein matrix.

4. A method according to Claim 3, wherein the basement membrane protein matrix is fibrin, collagen, or MATRIGEL®.

5. A method according to Claim 1, wherein the growth media includes serum.

6. A method according to Claim 1, wherein the loss of normal response comprises impaired angiogenesis.

7. A method according to Claim 1, wherein the endothelial cells are cultured in the presence of the cytokine for period of time sufficient to impair angiogenesis.

8. A method according to Claim 7, wherein the period of time is at least 3 days.

9. A method for identifying genes involved in endothelial cell dysfunction, said method comprising the steps of:
(a) culturing endothelial cells together with a physiological protein matrix, growth media including serum, and at least one proinflammatory cytokine for a period of time sufficient to disrupt normal endothelial function; and
(b) measuring expression of at least one selected gene, wherein altered expression of the at least one selected gene, relative to endothelial cells not contacted with the proinflammatory cytokine, indicates that the selected gene is involved in endothelial cell dysfunction.

10. A method according to Claim 9, wherein the endothelial cells are cultured in the presence of the cytokine for period of time sufficient to impair angiogenesis.

11. A method according to Claim 9, wherein the physiological protein matrix is a basement membrane protein matrix.

12. A method according to Claim 11, wherein the basement membrane protein matrix is fibrin, collagen, or MATRIGEL®.

13. A method for determining whether candidate compound is useful for modulating angiogenesis, said method comprising the steps of:
(a) culturing endothelial cells together with a physiological protein matrix, growth media including serum, at least one proinflammatory cytokine, and a candidate compound for a period of time sufficient to disrupt normal endothelial function; and
(b) measuring the angiogenic actively of the endothelial cells, wherein altered angiogenic activity, relative to endothelial cells treated as in Step (a) not contacted with the candidate compound, indicates that the candidate compound modulates angiogenesis.

14. A method according to Claim 13, wherein the angiogenic activity is growth of new vascular tissue.

15. A kit comprising, in compartmentalized form, at least one first compartment containing at least one physiological protein matrix for culturing endothelial cells thereon, at least one second compartment containing growth media including serum, and endothelial cells for addition to the at least one physiological protein matrix; a third compartment containing at least one proinflammatory cytokine for addition to either said first compartment of said second compartment.
